# EUROPÄISCHE PATENTANMELDUNG

(11) **EP 4 458 296 A1**
(43) Veröffentlichungstag der Anmeldung: **06.11.2024**
(21) Anmeldenummer: 23171481.7
(22) Anmeldetag: 04.05.2023
(51) Int. Cl.: A61B 18/00, A61B 18/14, A61B 18/04

(54) **PLASMAINSTRUMENT**

(71) Anmelder: Erbe Elektromedizin GmbH, 72072 Tübingen (DE)
(72) Erfinder: WALZ, Martin, 72108 Rottenburg (DE); MOSER, Sandra, 72459 Albstadt-Laufen (DE); HEYM, Johannes, 72074 Tuebingen (DE); BEUTLER, Fabian, 71654 Neckartenzlingen (DE)
(74) Vertreter: Rüger Abel Patentanwälte PartGmbB

(57) **Zusammenfassung**

Ein erfindungsgemäßes Instrument (11) zur Plasmabehandlung von biologischem Gewebe weist Plasmaaustrittsfenster in Gestalt von Öffnungen (17 - 22) auf, die sich in Längsrichtung erweitern oder verjüngen. Dadurch ergeben sich Schrägstellungen der zwischen den einzelnen Öffnungen vorhandenen Stege (23 - 28), die einen proximalen Abschnitt des Instrumentenkopfs (16) mit einem distalen Abschnitt einstückig nahtlos verbinden. Durch die Schrägstellung der Stege wird deren Abschattungswirkung minimiert oder beseitigt.

## Beschreibung

Die Erfindung betrifft ein Plasmainstrument zur Einwirkung auf biologisches Gewebe insbesondere zur chirurgischen Behandlung von menschlichem oder tierischem Gewebe. Das Plasmainstrument ist insbesondere als seitlich bzw. radial wirksames Instrument ausgebildet.

Aus der EP 1 682 023 B1 ist eine Plasmasonde mit einem schlauch- oder rohrartigen Grundkörper bekannt, durch den sich ein Lumen von dem proximalen Ende zu einem distalen Ende erstreckt. Das Lumen ist an eine Gasquelle angeschlossen. Durch das Lumen erstreckt sich ein elektrischer Leiter, der proximal an eine elektrische Quelle angeschlossen ist. Distal ragt er axial aus dem Lumen heraus. An seinem distalen Ende trägt der als Elektrode dienende Leiter einen zum Beispiel kugelförmigen oder tellerförmigen Isolatorkörper, der mit der Berandung der Mündung des Lumens einen Ringspalt festlegt. Durch diesen Ringspalt kann in jeder beliebigen radialen Richtung ein Plasmastrom abgehen, je nachdem, in welcher Richtung das nächstliegende, an den Gegenpol der elektrischen Quelle angeschlossene biologische Gewebe liegt.

Jede auf den von der Elektrode getragenen Isolatorkörper einwirkende Kraft muss von der Elektrode aufgenommen und auf das distale Ende des Schlauchs übertragen werden.

Es sind auch Sonden bekannt, bei denen an dem distalen Ende des Schlauchs ein Endstück aus Keramik mit einer oder mehreren seitlichen Öffnungen vorgesehen ist, aus denen jeweils ein Plasmastrahl austreten kann. Dabei zeigt sich aber, dass der Plasmastrahl je nachdem, wo das nächste biologische elektrisch leitfähige Gewebe liegt, zum Springen zwischen den Fenstern neigt.

Aus dem Stand der Technik sind weitere Plasmainstrumente zur Einwirkung auf biologisches Gewebe bekannt. Beispielsweise offenbaren die DE 198 20 240 A1 wie auch die EP 1 297 082 A1 Instrumente jeweils mit einem schlauchartigen Grundkörper, durch den sich ein Lumen von seinem proximalen Ende zu seinem distalen Ende erstreckt, an dem ein Kopf mit einem oder mehreren seitlichen Fenstern angeordnet ist. Durch das Lumen verläuft ein elektrischer Leiter, der an seinem distalen Ende mit einer zentralen Elektrode verbunden ist. In Betrieb ionisiert die an eine HF-Spannungsquelle angeschlossene Elektrode durch das Lumen heranströmendes Gas, das dann als Plasmastrom seitlich austritt. In einer Ausführungsform sind zwei schlitzartige Plasmaaustrittsfenster vorgesehen, die an axial unterschiedlichen Stellen angeordnet sind und einander etwas überlappen.

Auch die EP 3 831 291 A1 offenbart ein Instrument zur Erzeugung eines Plasmas zur Einwirkung auf biologisches Gewebe. Wiederum weist das Instrument einen schlauchartigen Körper auf, der an seinem distalen Ende zumindest in einer Ausführungsform einen Kopf mit einer seitlichen Öffnung trägt, durch die ein Plasmastrom austreten kann.

Das aus der US 9 510 889 B2 bekannte Instrument weist an seinem distalen Ende eine axiale Öffnung auf, aus der eine Elektrode ragt. Die Elektrode trägt an ihrem distalen Ende einen Isolierkörper, der mit dem übrigen Instrument einen ringförmigen, sich radial öffnenden Schlitz begrenzt. Der Plasmastrom hat radial an jeder Umfangsstelle 360° freien Austritt, wobei aber der Isolierkörper von der Elektrode getragen und stabil gehalten werden muss.

Weiterer Stand der Technik kann der US 2021/259 756 A1**,** der EP 3 422 981 A2, der EP 3 372 183 A1**,** der JP 2002-2301088 A und der GB 2 573 128 A entnommen werden.

Es ist Aufgabe der Erfindung, eine robuste Plasmasonde mit gleichmäßiger radialer Wirksamkeit zu schaffen.

Diese Aufgabe wird mit der Sonde gemäß Anspruch 1 gelöst:

Das erfindungsgemäße Plasmainstrument ist insbesondere als Sonde für den endoskopischen Anwendungsfall ausgebildet. Es kann jedoch bedarfsweise auch als laparoskopisches Instrument oder als Instrument für den offenchirurgischen Einsatz ausgebildet sein.

Das Instrument weist einen länglichen als Schlauch oder Rohr ausgebildeten Körper auf, durch den sich längs mindestens ein Lumen erstreckt. Der Körper kann ein oder mehrere Lumen aufweisen, also ein ein- oder mehrlumiger Schlauch sein. Er kann auch als ein- oder mehrlumiges Rohr ausgebildet sein.

Das proximale Ende des Körpers bzw. seines Lumens ist an eine Gasquelle, beispielsweise ein entsprechendes Gerät, anschließbar, über das das Instrument mit einem Gas, insbesondere einem Inertgas, wie beispielsweise Argon versorgbar ist. Das Gerät oder die sonstige Gasquelle ist darauf eingerichtet, das Gas für das Instrument mit dem erforderlichen Druck und in der erforderlichen Menge bereitzustellen.

An dem distalen Ende des Körpers ist ein Kopf vorgesehen, der wenigstens zwei mit dem oder den Lumen verbundene Öffnungen aufweist, die voneinander durch einen Steg getrennt sind. Der Steg ist schräg, d.h. nicht parallel zu der Längsrichtung des Instruments ausgerichtet. Durch die Schrägstellung des Steges kann jede Öffnung an ihrem distalen oder an ihrem proximalen Ende unterschiedlich lange Kanten aufweisen. Für jeden Steg gilt, dass das sein distales Ende in Umfangsrichtung gegen sein proximales Ende versetzt ist. Die Umfangsrichtung wird dabei durch einen Kreis um die Längsmittelachse des Kopfs (und Schlauchs) definiert.

Vorzugsweise sind die Öffnungen trapez- oder dreieckförmig. Dabei ist weiter vorzugsweise die Summe der langen Kanten aller Fenster größer als es bei längsorientierten Stegen der Fall wäre. Es existieren Radialrichtungen, die beiden Öffnungen gemeinsam sind. Dadurch kann ein radial austretender Plasmastrahl weniger sprunghaft und somit in einem weicheren Wechsel von einem Fenster zu einem anderen übergehen. Das Verhalten des erfindungsgemäßen Instruments hinsichtlich der Einflussnahme auf den Einwirkungsort des Plasmastrahls ist für den Behandler deswegen besser vorhersehbar und leichter zu steuern.

Bei einer bevorzugten Ausführungsform ist die Summe der langen Kanten der Fenster größer als der Umfang des Kopfs. Bei einer bevorzugten Ausführungsform gilt dies, wenn bei einem sich in distaler Richtung verjüngenden Kopf der Umfang an dem distalen Ende der Fenster gemessen wird. Damit wird eine große Überlappung der Öffnungen erreicht, wodurch ein besonders weicher Plasmaübergang erreicht wird.

Nach dem erfindungsgemäßen Konzept ist der Steg in Umfangsrichtung geneigt. Dabei können mehrere Stege jeweils gegensinnig geneigt sein, sodass sich abwechselnd in distaler Richtung sich verjüngende und in distaler Richtung sich erweiternde Öffnungen bilden, die entlang des Umfangs des Kopfs in einer Reihe angeordnet sind. Dabei können alle

Öffnungen proximal an einer einheitlichen Axialposition enden. Auch können die distalen Begrenzungen aller Fenster an der jeweils gleichen Axialposition lokalisiert sein. Die Flächenschwerpunkte der einzelnen Öffnungen können dann auf einer Zickzacklinie liegen.

Vorzugsweise ist die Schrägstellung des Stegs in Umfangsrichtung so groß, dass die Öffnungen einander axial und in Umfangsrichtung etwas überlappen. Der Übergang des Plasmastrahls oder Funkens von einer Öffnung zur anderen ist dann besonderes stetig.

Die Öffnungen können dreieckförmig, dreieckförmig mit gerundeten Ecken, trapezförmig, vorzugsweise mit gerundeten Ecken, oder auch rautenförmig ausgebildet sein. In allen diesen Fällen ist die Behinderung des Plasmastrahls oder Funkens durch die Stege weitgehend minimiert.

Der Plasmastrahl kann prinzipiell auf unterschiedliche Weise erzeugt werden. Zum Beispiel kann in dem Lumen in der Nähe der Öffnung eine Elektrode angeordnet sein, die über eine elektrische Leitung mit einer elektrischen Quelle verbunden ist. Die Elektrode kann konzentrisch an dem Kopf angeordnet sein und somit auf seiner Längsmittelachse liegen. Die elektrische Leitung kann sich durch das Lumen erstrecken oder in das Material des Körpers (Schlauchs) eingearbeitet sein. Die elektrische Leitung ist darauf eingerichtet, an ihrem proximalen Ende mit einem Pol eines elektrischen Generators verbunden zu werden, um die Elektrode mit Strom und Spannung zu versorgen. Vorzugsweise ist der Generator ein Hochfrequenzgenerator. Ist nur eine Elektrode vorhanden, handelt es sich um ein monopolares Instrument, bei dem die Gegenelektrode der Spannungsquelle an den Patienten angeschlossen werden muss. Der Stromfluss geht dann von der Elektrode durch das die Öffnung verlassende Plasma zu dem Gewebe.

Alternativ kann das Plasma auch auf andere Weise erzeugt werden. Beispielsweise kann dazu zur Generierung eines nichtthermischen Plasmas wenigstens eine elektrisch isolierte Elektrode vorgesehen sein, die zum Beispiel in den Kopf eingelassen sein kann. Eine weitere Elektrode kann ebenfalls in den Kopf eingelassen oder in dem Lumen angeordnet sein. Solche Elektroden können über Leitungen mit den Polen eines elektrischen Hochfrequenzgenerators verbunden sein, um in dem Kopf eine sogenannte Barriereentladung zu erzeugen.

Weitere Einzelheiten vorteilhafter Ausführungsformen der Erfindung sind Gegenstand von Unteransprüchen oder der Beschreibung sowie der Zeichnung. In der Zeichnung zeigen:
Figur 1 eine Ausführungsform eines erfindungsgemäßen Plasmainstruments, angeschlossen an ein speisendes Gerät, in perspektivischer Prinzipdarstellung,
Figur 2 das distale Ende des Plasmainstruments nach Figur 1 in vergrößerter, dabei aber nicht maßstäblicher Seitenansicht,
Figur 3 das Plasmainstrument nach Figur 1 und 2, geschnitten entlang der Schnittlinie III-III in Figur 2,
Figur 4 das Plasmainstrument nach Figur 2 und 3, geschnitten entlang der Schnittlinie III-III, ausschnittsweise und stark vergrößert,
Figur 5 eine Abwicklung des aus Figur 2 ersichtlichen Kopfs zur Veranschaulichung der geometrischen Verhältnisse,
Figur 6 eine abgewandelte Ausführungsform eines erfindungsgemäßen Plasmainstruments, in geschnittener, ausschnittsweiser Perspektivdarstellung,
Figur 7 eine Abwicklung des Kopfs nach Figur 6 zur Veranschaulichung der geometrischen Verhältnisse,
Figur 8 und 9 Abwicklungen weiterer alternativer Ausführungsformen erfindungsgemäßer Sonden und
Figur 10 eine Plasmasonde mit wenigstens zwei Elektroden, in ausschnittsweiser und teilweise geschnittener Darstellung ihres Kopfs.

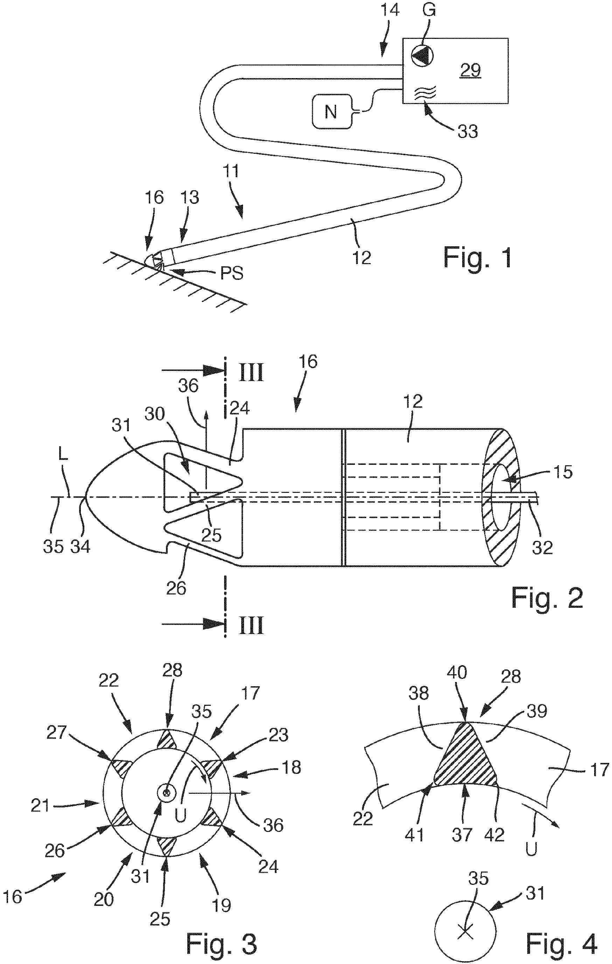

Figur 1 veranschaulicht ein als flexible Sonde ausgebildetes Plasmainstrument 11, das einen flexiblen Schlauch als Körper 12 aufweist. Der Körper 12 weist ein distales Ende 13 und ein proximales Ende 14 sowie ein Lumen 15 auf, das sich von dem proximalen Ende 14 zu dem distalen Ende 13 erstreckt. Der Körper 12 kann, wie in Figur 2 dargestellt, ein einziges Lumen 15 oder auch mehrere Lumen aufweisen. Der Körper 12 ist biegsam oder flexibel ausgebildet, um beispielsweise als Sonde durch den Arbeitskanal eines Endoskops an den Operationsort eines Patienten geschoben zu werden. Wenn das Endoskop ein steuerbares Ende aufweist, gestattet die Flexibilität des Instruments die Durchführung einer entsprechenden Abwinkelbewegung.

Das Instrument 11 kann nicht nur als endoskopisch nutzbare Sonde, sondern auch in anderer Form bereitgestellt werden, beispielsweise als laparoskopisches Instrument, wobei dann der Körper 12 ein starres Rohr ist. Auch kann das Instrument 11 für den offenchirurgischen Einsatz mit kurzem Körper 12 und am proximalen Ende vorgesehenen Handstück ausgebildet sein.

An dem distalen Ende 13 des Körpers 12 ist ein Kopf 16 angeordnet, der wenigstens zwei, vorzugsweise aber mehrere, wie aus Figur 3 hervorgeht, beispielsweise sechs Öffnungen 17 - 22 aufweist. Alternativ können auch drei, vier, fünf, sieben, acht oder mehr Öffnungen vorgesehen sein. Die Öffnungen 17 - 22 sind untereinander durch Stege 23 - 28 getrennt, die, wie auch vorzugsweise der gesamte Kopf 16, aus temperaturstabilem Material, zum Beispiel Keramik bestehen.

Das Lumen 15 ist an dem proximalen Ende 14 an ein Gerät 29 angeschlossen, das zur Versorgung und Speisung des Instruments 11 dient. Zum Beispiel kann das Gerät 29 als Gasquelle G ausgebildet sein oder eine solche enthalten. Zu diesem Zweck kann es an einen Gasspeicher, beispielsweise eine Gasflasche oder dergleichen, angeschlossen sein und Mittel zum Steuern, insbesondere zum Freigeben und Sperren, sowie gegebenenfalls zum Dosieren des Gasflusses enthalten, mit dem das Lumen 15 gespeist wird. Der Gasspeicher kann insbesondere Argon enthalten, oder auch ein anderes Inertgas, insbesondere ein zur Plasmabildung geeignetes Inertgas. Bedarfsweise kann die Sonde 29 auch zur Lieferung von aktiven Gasen, d.h. reaktiven Gasen, Aerosolen, Dämpfen oder dergleichen ausgebildet sein.

Das Lumen 15 ist mit den Öffnungen 17 bis 22 verbunden, sodass durch das Lumen zu dem Kopf 16 herangeführtes Gas aus allen Öffnungen 17 bis 22 gleichberechtigt austreten kann.

Das Instrument 11 enthält außerdem mindestens eine Plasmaerzeugungseinrichtung 30, zum Beispiel in Gestalt einer Elektrode 31, deren distales Ende im Bereich der Öffnungen 17 bis 22 liegt. Die Elektrode kann durch einen im Wesentlichen zylindrischen Körper aus Metall gebildet sein, dessen distales Ende im Bereich der Öffnungen 17 bis 22, vorzugsweise etwa in der Mitte derselben, angeordnet ist. Die Elektrode 31 kann wie dargestellt einen Kreisquerschnitt oder auch einen Polygonalquerschnitt aufweisen. Anstelle einer einzelnen Elektrode kann auch ein Bündel von elektrischen Leitern vorgesehen sein, die einander berühren oder in seitlichen Abständen zueinander angeordnet sind. Vorzugsweise, aber nicht zwingend, weist die Elektrode 31 einen Durchmesser auf, der mindestens so groß ist wie eine in der Schnittebene nach Figur 3 und 4 in Umfangsrichtung U gemessene Breite eines Stegs. Im Falle eines Elektrodenbündels ist der Durchmesesr D der Außendurchmesser des Querschnitts des Bündels.

Von der Elektrode 31 kann sich ein elektrischer Leiter 32 bis zu dem proximalen Ende 14 des Instruments 11 erstrecken und dort an das Gerät 29 angeschlossen sein. Das Gerät 29 kann eine elektrische Quelle, beispielsweise in Gestalt eines Hochspannungs-Hochfrequenz-Generators 33 enthalten, über den die Elektrode 31 mit einer elektrischen Spannung speisbar ist. Die hochfrequente Spannung hat einen derartigen Wert, dass sie zur Ionisation des über das Lumen 15 herangeführten Gasstroms ausreicht, um ein Plasma zu bilden. Die Spannung beträgt üblicherweise mehrere 100 Volt bei einer Frequenz oberhalb 100 kHz, vorzugsweise mehrere 100 kHz, weiter vorzugweise jedoch unter 5 MHz.

Die Besonderheit der Erfindung liegt in der Ausbildung des Kopfs 16, der zumindest vorzugsweise einen sich zu der distalen Spitze 34 hin verjüngenden Querschnitt aufweist. Die Öffnungen 17 - 22 und somit auch die Stege 23 - 28 können ganz oder teilweise in dem sich verjüngenden Bereich des Kopfs 16 angeordnet sein, welcher in Figur 2 etwa bei der Schnittlinie III - III beginnt. In dem sich verjüngenden Bereich nimmt der Durchmesser des Kopfs 16 kontinuierlich bis zu der distalen Spitze 34 hin ab, wo der Kopf 16 in einer Rundung vorzugsweise stumpf endet.

Die einzelnen Öffnungen 17 bis 22 sind voneinander durch die Stege 23 - 28 getrennt, die gegenüber einer Längsrichtung L zumindest in Umfangsrichtung geneigt angeordnet sind. Die Längsrichtung L ist in Figur 2 durch eine Längsmittelachse 35 angegeben, die eine Symmetrieachse für eine Drehsymmetrie des Kopfs 16 bildet. Die Drehsymmetrie ist in dem Ausführungsbeispiel nach Figur 2 und 3 dreifach, d.h. bei einer Drehung des Kopfs um 120° um die Längsmittelachse 35 ist der gedrehte Kopf 16 deckungsgleich mit dem nicht gedrehten Kopf 16.

Die zwischen den Stegen 23 - 28 begrenzten Öffnungen 17 bis 22 sind radial zu der Längsmittelachse 35 orientiert. In Figur 2 ist die Öffnungsrichtung der Öffnung17 durch einen gestrichelten Pfeil 36 markiert. Die Öffnungen 17 bis 22 öffnen sich demnach quer zu der Längsmittelachse 35 und somit auch quer zur der Längsrichtung L in radialer Richtung oder, wie durch den Pfeil 36 angedeutet, auch gegen die Radialrichtung etwas geneigt. Zur Bestimmung der Öffnungsrichtung kann der Kopf 16 zunächst ohne Fenster als sich zu der distalen Spitze 34 hin verjüngender Rotationskörper gedacht werden. Die Verjüngung kann, wie Figur 2 veranschaulicht, zu der distalen Spitze 34 hin zunehmen, so dass sich der Längsschnitt durch den Kopf 16 zum Beispiel an eine Parabelform oder eine Halbellipse annähert. Wird auf einem solchen noch öffnungslos gedachten Kopf 16 an der für die jeweilige Öffnung 17 bis 22 vorgesehenen Stelle eine Öffnung aufgezeichnet und im Flächenschwerpunkt derselben der Normalenvektor angetragen, bezeichnet dieser die Richtung des Pfeils 36.

Die Stege 23 bis 28 weisen vorzugsweise einen sich in positiver Radialrichtung +R (radial nach außen) verjüngenden Querschnitt, z.B. einen dreieckigen Querschnitt auf, der in Figur 4 am Beispiel des Stegs 28 veranschaulicht ist. Dieser ist in der gleichen Schnittebene III-III geschnitten, wie in Figur 2 eingezeichnet und in Figur 3 dargestellt.

Der in Figur 4 veranschaulichte Querschnitt des Stegs 28 weist eine der Längsmittelachse 35 zugewandte Seitenfläche 37 sowie zwei weitere Seitenflächen 38, 39 auf, die die Fenster 22 und 17 seitlich begrenzen. Die Querschnitte der Stege 23 - 28 sind entlang jedes Steges vorzugsweise im Wesentlichen unverändert. Die ebenen oder auch konvex gerundet ausgebildeten Seitenflächen 38, 39 schlie-βen miteinander einen spitzen Winkel ein, der sich zu der Längsmittelachse 35 hin öffnet. Damit weist der Steg 28 mit seiner innen liegenden Seitenfläche 37 auf die Elektrode 31. Mit seiner vorzugsweise gerundeten Ecke 40 weist der Steg 28 von der Längsmittelachse 35 gesehen radial nach au-βen, d.h. von der Längsmittelachse 35 weg. Die übrigen Ecken 41, 42 sind vorzugsweise ebenfalls gerundet. Die Seitenflächen 38, 39 können eben oder vorzugsweise etwas konvex gerundet sein. Die Seitenfläche 37 kann eben oder, wie in Figur 4 dargestellt, konkav, bedarfsweise aber auch konvex gerundet sein.

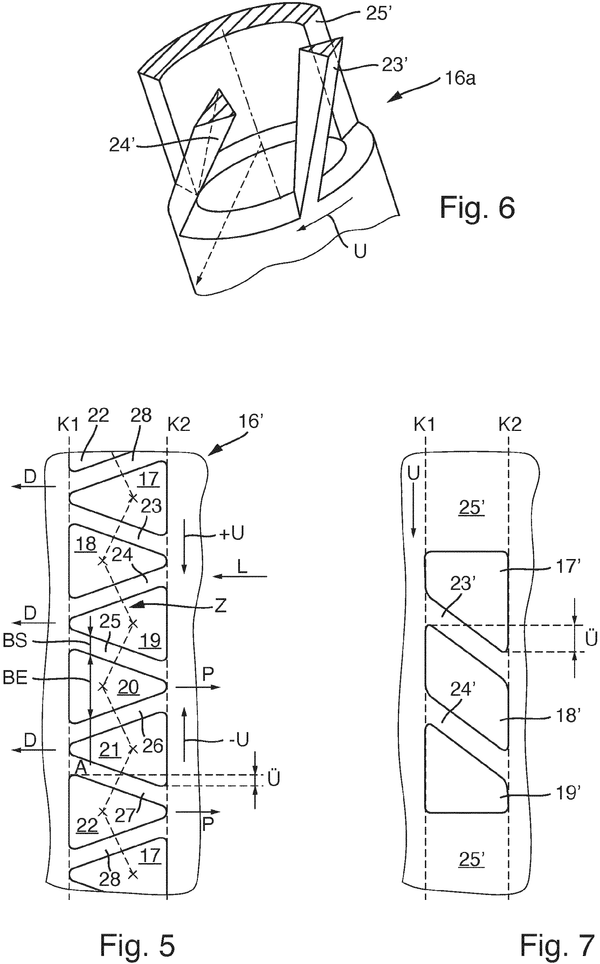

Figur 5 veranschaulicht eine Abwicklung 16' des Kopfs 16, deren Enden sich überlappen. Die sechs Fenster 17 bis 22 sind wie ersichtlich dreieckig. Benachbarte Stege 23 bis 28 sind jeweils paarweise gegensinnig in Umfangsrichtung U geneigt. Dabei stimmen die Beträge der Neigungswinkel, den die einzelnen Stege 23 bis 28 mit der Umfangsrichtung U einschließen, miteinander überein. Mit anderen Worten, der Neigungswinkel jedes Stegs 23 bis 28 wechselt im Verlauf der Umfangsrichtung von Steg zu Steg jeweils sein Vorzeichen. Dadurch erhalten die Öffnungen 17 bis 22 jeweils eine dreieckige Grundform mit mehr oder weniger stark gerundeten Ecken, wobei die Basen der durch die Öffnungen 17 bis 22 markierten Dreiecke auf zwei zueinander parallelen Kreisen K1, K2 gleichen oder leicht unterschiedlichen Durchmessers liegen. Die Mittelpunkte der Kreise K1 und K2 liegen auf der Längsmittelachse 35. Die Spitzen der durch die Öffnungen 17 bis 22 markierten Dreiecke weisen abwechselnd in distaler Richtung D oder proximaler Richtung P. Die wesentlichen dreieckigen Öffnungen 17 bis 22 weisen Flächenschwerpunkte auf, die wie in Figur 5 angedeutet auf einer Zick-Zack-Linie Z liegen können.

Die Neigungen bzw. Neigungswinkel der Stege 23 bis 28 sind so bemessen, dass sich die Öffnungen 17 bis 22 in Umfangsrichtung U überlappen. Die Umfangsrichtung ist durch den Verlauf der hier durch die Abwicklung als Geraden veranschaulichten Kreise K1, K2 gekennzeichnet. In Figur 5 ist die Überlappung am Beispiel des Stegs 27 und den Öffnungen 21, 22 veranschaulicht. Die in positiver Umfangsrichtung +U vorn liegende Ecke der Öffnung 21 und die in positiver Umfangsrichtung +U hinten liegende Öffnung der Ecke 22 überlappen einander um den Überlappungsbetrag Ü. Dieser ist mindestens Null und vorzugsweise größer als Null. Mit anderen Worten, wird die Öffnung 21 gedanklich in distaler Richtung D und/oder die Öffnung 22 gedanklich in proximaler Richtung P verschoben, berühren oder überlagern die Öffnungen 21, 22 einander.

Jeder Steg weist eine in Umfangsrichtung U zu messende Breite BS und jede Öffnung weist eine in Umfangsrichtung zu messende Breite BE auf. Figur 5 veranschaulicht dies anhand des Stegs 25 und der Öffnung 20 an einer Axialposition A, die dem breiten Ende der Öffnung 20 und somit dem Kreis K1 benachbart liegt. Für die Öffnung 19 gilt dies entsprechend mit dem Kreis K2. Dies bedeutet, dass die Breite BS des Stegs 23 vorzugsweise geringer als die Breite BE zumindest einer benachbarten Öffnung ist. Dies gilt zumindest an einer Axialposition, wobei die Breite BS und die Breite BE an der gleichen Axialposition A gemessen werden. Die genannte Bedingung für die Breiten BS und BE (BE>BS) gilt jeweils mindestens dort, wo die Öffnungen ihre größte Ausdehnung in Umfangsrichtung U haben.

Bei dem Ausführungsbeispiel nach Figur 1 bis 5 sind die Stege 23 bis 28 paarweise gegensinnig in und gegen die Umfangsrichtung (d.h. in +U und -U) geneigt. Es ist jedoch auch möglich, Stege 23', 24` gleichsinnig zu neigen, wie anhand eines Ausführungsbeispiels nach den Figuren 6 und 7 veranschaulicht ist. Während es sich bei dem vorbeschriebenen Instrument 11 um ein Instrument mit 360° Rundumwirksamkeit handelt, ist das Instrument 11` nach Figur 6 zur Gewebebehandlung lediglich in einem eingeschränkten Umfangsbereich vorgesehen. Der Kopf 16a weist einen breiteren Abschnitt 25` auf, der mit oder ohne Neigung in Umfangsrichtung ausgebildet ist und als gebogene Wand, nicht aber als Steg gedacht werden kann. Es ergeben sich drei Öffnungen 17`, 18`, 19`, die trapezförmig und rautenförmig ausgebildet sein können. Wie ersichtlich überlappen sich wiederum die trapezförmige Öffnung 17' und die rautenförmige Öffnung 18' in Umfangsrichtung U. Gleiches gilt für die Öffnung 18' und die trapezförmige Öffnung 19`. Im Übrigen gilt die vorige Beschreibung unter Zugrundelegung der bereits eingeführten Bezugszeichen entsprechend.

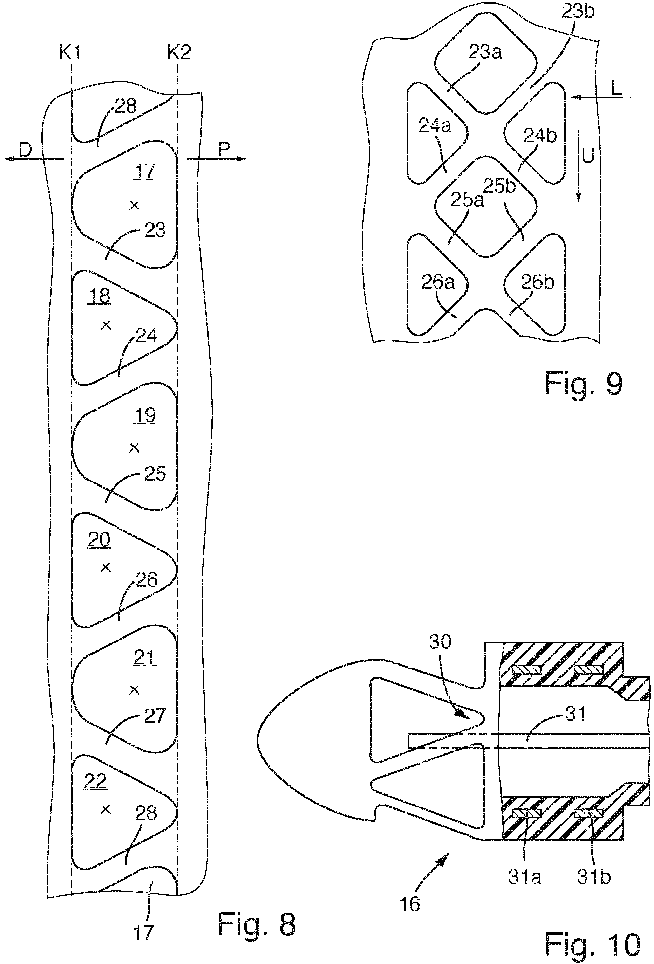

Weitere Abwandlungen sind möglich. Figur 8 zeigt dazu eine Abwicklung eines modifizierten Kopfs 16, für den die Beschreibung der Ausführungsform nach den Figuren 1 bis 5 mit Ausnahme der nachfolgend genannten Besonderheiten vollständig entsprechend gilt:

Die Öffnungen 17 - 22 sind nicht dreieck- sondern trapezförmig ausgebildet. Dazu ist anstelle der jeweiligen in distaler Richtung D oder proximaler Richtung P weisenden Spitze jeder Öffnung 17 bis 22 eine starke Rundung oder auch eine kurze Kante vorgesehen, die über zwei Rundungen in den jeweiligen Steg 23 bis 28 übergeht.

Die bislang beschriebenen Ausführungsformen nutzen Öffnungen 17 bis 22, die zwischen den beiden gedachten Kreisen K1, K2 in einer einzigen Reihe angeordnet sind. Es sind jedoch auch mehrreihige Anordnungen möglich, wie Figur 9 veranschaulicht. Die Besonderheit dieser aus insgesamt drei Reihen gerundeter drei- oder viereckiger Öffnungen bestehenden Anordnung sind wiederum geneigte Stege 23a, 23b, 24a, 24b, 25a, 25b, usw., die in der oder gegen die Umfangsrichtung U geneigt und somit nichtparallel zu der Längsrichtung L angeordnet sind. Wiederum überlappen die einzelnen Öffnungen in Umfangsrichtung einander.

Eine weitere Abwandlung ist hinsichtlich der Ausbildung der Plasmaerzeugungseinrichtung 30 möglich. Während bei den bislang beschriebenen Ausführungsformen als Plasmaerzeugungseinrichtung 30 lediglich eine blanke Elektrode 31 vorgesehen worden ist, um ein weitgehend thermisches Plasma zu erzeugen, können alternativ auch ein oder mehrere elektrisch isoliert angeordnete Elektroden 31a, 31b zur Ausbildung einer Barriereentladung vorgesehen sein. Beispielsweise können die Elektrode 31a und/oder die Elektrode 31b als schalen- oder ringförmige Elektroden in das Material des Kopfs 16 eingebettet oder auf andere Weise isoliert in diesem angeordnet sein. Die beiden Pole des Generators können mit den isolierten Elektroden 31a, 31b verbunden sein, um eine Barriereentladung zu erzeugen. In diesem Fall kann die Elektrode 31 entfallen. Alternativ können eine oder beide Elektroden 31a, 31b mit einem Pol des Generators und die mit einer Isolierschicht versehene oder blanke Elektrode 31 mit dem anderen Pol des Generators verbunden sein, um eine Barriereentladung zu bilden. Solche Anordnungen dienen insbesondere zur Erzeugung von nicht thermischem warmem oder kaltem Plasma. Die verschiedenen hier kurz beschriebenen Plasmaerzeugungseinrichtungen 30 können mit jedem der vorstehend beschriebenen Köpfe nach Figur 1 bis 9 kombiniert werden.

Das insoweit beschriebene Instrument 11 arbeitet wie folgt:
In Betrieb wird das Instrument 11 von dem Gerät 29 mit Gas, beispielsweise Argon versorgt, so dass dieses durch das Lumen 15 hindurch und aus den Öffnungen 17 - 22 ausströmt. Außerdem wird die elektrische Quelle 33 aktiviert, so dass an der Plasmaerzeugungseinrichtung 30 eine elektrische Entladung zur Ionisierung des Gasstroms und zur Erzeugung von Plasma stattfindet. Insbesondere in der monopolaren Variante, bei der ein Pol der elektrischen Quelle 33 mit der vorzugsweise nackten Elektrode 31, und der andere Pol der Quelle 33 mit dem Patienten verbunden ist, bildet sich eine elektrische Entladung zwischen der Elektrode 31 und dem Kopf 16 nächstgelegenen biologischem Gewebe, so dass sich ein Plasmastrom PS durch die dazwischenliegende Öffnung, zum Beispiel die Öffnung 19 etabliert. Dieser Zustand ist in Figur 1 veranschaulicht.

Wird nun das Instrument 11 bewegt, kann es sein, dass die Entladung, das heißt der Plasmastrom, seinen Weg eher durch das benachbarte Fenster 18 oder 20 sucht. Der Übergang des Plasmastroms von dem Fenster 19 zu dem Fenster 18 oder 20 ist dabei kontinuierlich und glatt, weil die Fenster, wie am Beispiel aus Figur 5 ersichtlich, einander insbesondere bei Blickrichtung in Längsrichtung L in Umfangsrichtung überlappen. Die schräg gestellten Stege 23 - 28 sind der Entladung somit nicht im Weg, was die Sprunghaftigkeit des vom Behandler als Funken wahrgenommenen Plasmastrahls gegenüber Instrumenten mit in Längsrichtung orientierten Stegen wesentlich reduziert.

Wird als Plasmaerzeugungseinrichtung 30 eine Vorrichtung mit Barriereentladung wie gemäß Figur 10 veranschaulicht genutzt, kann erreicht werden, dass das Plasma in einem alle Öffnungen 17 bis 22 lückenlos einnehmenden ringförmigen Bereich entsteht, wobei Abschattungen durch die Stege 23 bis 28 wiederum minimiert sind.

Ein erfindungsgemäßes Instrument 11 zur Plasmabehandlung von biologischem Gewebe weist Plasmaaustrittsfenster in Gestalt von Öffnungen 17 - 22 auf, die sich in Längsrichtung erweitern oder verjüngen. Jede Öffnung 17 - 22 weist mindestens einen zur Längsrichtung L schräg verlaufenden Rand auf, der durch eine Flanke 38, 39 eines Steges gebildet sein kann. Es ergeben sich Schrägstellungen der zwischen den einzelnen Öffnungen vorhandenen Stege 23 bis 28, die einen proximalen Abschnitt des Instrumentenkopfs 16 mit einem distalen Abschnitt einstückig nahtlos verbinden. Durch die Schrägstellung der Stege wird deren Abschattungswirkung minimiert oder beseitigt.

### Bezugszeichen:

- 11: Instrument
- 12: Körper
- 13: distales Ende des Körpers
- 14: proximales Ende des Körpers
- 15: Lumen
- 16: Kopf
- 16': Abwicklung des Kopfs 16
- 17 - 22: Öffnungen
- 23 - 28: Stege
- 29: Gerät
- 30: Plasmaerzeugungseinrichtung
- 31: Elektrode
- 32: Leiter
- G: Gasquelle
- 33: elektrische Quelle
- 34: distale Spitze des Kopfs 16
- L: Längsrichtung
- 35: Längsmittelachse
- 36: Pfeil der Öffnungsrichtung
- 37: Innere Seitenfläche des Stegs 28
- 38, 39: Seitenflächen an den Fensterflanken
- 40 - 42: Ecken des Stegquerschnitts
- K1, K2: Kreise
- U: Umfangsrichtung
- Ü: Überlappungsbetrag
- D: distale Richtung
- P: proximale Richtung
- Z: Zickzacklinie
- PS: Plasmastrom

## Patentansprüche

1. Plasmainstrument (11) zur Einwirkung auf biologisches Gewebe, insbesondere zur chirurgischen Behandlung von menschlichen oder tierischen Patienten,
mit einem länglichen, als Schlauch oder Rohr ausgebildeten Körper (11), der ein distales Ende (13) und ein proximales Ende (14) sowie ein eine Längsrichtung (L) festlegendes Lumen (15) aufweist, das sich von dem proximalen Ende (14) bis zu dem distalen Ende (13) erstreckt und an eine Gasquelle (G) angeschlossen oder anschließbar ist,
mit einem an das distale Ende (13) angeschlossenen Kopf (16), der wenigstens zwei mit dem Lumen (15) verbundene Öffnungen (17, 18) aufweist, die voneinander durch einen Steg (23) getrennt sind, der schräg zu der Längsrichtung (L) ausgerichtet ist.

2. Plasmainstrument nach Anspruch 1, **dadurch gekennzeichnet, dass** in dem Lumen (15) und/oder in dem Kopf (16) eine Plasmaerzeugungseinrichtung (30) angeordnet ist.

3. Plasmainstrument nach Anspruch 2, **dadurch gekennzeichnet, dass** zu der Plasmaerzeugungseinrichtung (30) wenigstens eine Elektrode (31, 31a, 31b) gehört, die über eine elektrische Leitung (32) mit einer elektrischen Quelle (33) verbindbar ist.

4. Plasmainstrument nach Anspruch 2, **dadurch gekennzeichnet, dass** die Elektrode (31) eine metallische, nicht isolierte Elektrode ist, die mit einem Pol der elektrischen Quelle (33) verbindbar ist, wobei der andere Pol der elektrischen Quelle (33) mit einer am Patienten zu befestigenden Neutralelektrode (N) verbindbar ist.

5. Plasmainstrument nach einem der vorstehenden Ansprüche, **dadurch gekennzeichnet, dass** die Öffnungen (17, 18) quer zu der Längsrichtung (L) orientiert sind.

6. Plasmainstrument nach einem der vorstehenden Ansprüche, **dadurch gekennzeichnet, dass** die Öffnungen (17, 18) zu der Längsrichtung (L) radial orientiert sind.

7. Plasmainstrument nach einem der vorstehenden Ansprüche, **dadurch gekennzeichnet, dass** der Steg (23) in einem sich distal verjüngenden Abschnitt des Kopfs (16) angeordnet ist.

8. Plasmainstrument nach einem der vorstehenden Ansprüche, **dadurch gekennzeichnet, dass** der Steg (23) in Umfangsrichtung (U) des Kopfs (16) geneigt ist.

9. Plasmainstrument nach Anspruch 8, **dadurch gekennzeichnet, dass** der Steg (23), zusätzlich zu der Schrägstellung in Umfangsrichtung (U), distal zu einer Längsmittelachse (35) hin geneigt ist, die in dem Kopf (16) mittig in Längsrichtung (L) verläuft.

10. Plasmainstrument nach einem der vorstehenden Ansprüche, **dadurch gekennzeichnet, dass** der Steg (23) derart schräg zu der Längsrichtung (L) orientiert ist, dass die Öffnungen (17, 18) mit Blickrichtung in Längsrichtung (L) aneinander anschließen oder einander überlappen.

11. Plasmainstrument nach einem der vorstehenden Ansprüche, **dadurch gekennzeichnet, dass** der Kopf (16) mehrere Stege (23 - 28) aufweist, die zwischen den Öffnungen (17 - 22) angeordnet sind.

12. Plasmainstrument nach Anspruch 11, **dadurch gekennzeichnet, dass** benachbarte Stege (17 - 22) paarweise in einander entgegengesetzten Richtungen (+U, -U) geneigt angeordnet sind.

13. Plasmainstrument nach einem der vorstehenden Ansprüche, **dadurch gekennzeichnet, dass** die Öffnungen (17 - 23) dreieckförmig mit gerundeten Ecken (40, 41, 42) ausgebildet sind.

14. Plasmainstrument nach einem der vorstehenden Ansprüche 1 - 11, **dadurch gekennzeichnet, dass** die Öffnungen (17 - 22) trapezförmig mit gerundeten Ecken ausgebildet sind.

15. Plasmainstrument nach einem der vorstehenden Ansprüche, **dadurch gekennzeichnet, dass** die Öffnungen (17 - 22) eine sich um den gesamten Umfang des Kopfs (16) erstreckende Reihe bilden.
